# EUROPEAN PATENT SPECIFICATION

(11) **EP 2 598 104 B1**
(45) Date of publication and mention of the grant of the patent: **18.12.2019**
(21) Application number: 11738709.2
(22) Date of filing: 29.07.2011
(51) Int. Cl.: A61K 8/02, A61K 8/97, A61K 8/99, A61Q 19/02, A61Q 19/08, A61K 8/66

(54) **USE OF GREEN COFFEE AND PROBIOTIC FOR REGULATING SKIN PIGMENTATION**
VERWENDUNG VON GRÜNEN KAFFEEBOHNEN UND PROBIOTIKA ZUR REGULIERUNG DER HAUTPIGMENTIERUNG
UTILISATION DES GRAINS DE CAFÉ VERTES ET DES PRODUITS PROBIOTIQUES POUR LA RÉGULATION DE LA PIGMENTATION DE LA PEAU

(30) Priority: 30.07.2010 EP 10171380
(43) Date of publication of application: 05.06.2013
(73) Proprietor: Société des Produits Nestlé S.A., 1800 Vevey (CH); L'Oréal, 75008 Paris (FR)
(72) Inventor: KRAEHENBUEHL, Karin, CH-1926 Fully (CH); MAUROUX, Olivier, CH-1543 Grandcour (CH); BEL RHLID, Rachid, CH-1073 Savigny (CH); MOODYCLIFFE, Angus, CH-1073 Savigny (CH); DIONISI, Fabiola, CH-1066 Epalinges (CH); GUITARD, Marjorie, CH-1073 Savigny (CH)
(74) Representative: Nony
(86) International application number: PCT/EP2011/063059
(87) International publication number: WO 2012/013762

(56) References cited:
- EP-A1- 1 674 106
- WO-A1-2004/026287
- US-A1- 2006 018 986
- US-A1- 2009 169 651

## Description

The present invention relates generally to the field of food supplements and/or food products for cosmetic purpose. More specifically, the present invention aims to provide a composition containing green coffee beans or an aqueous extract thereof and *Lactobacillus johnsonii* as an active agent for treating and/or preventing skin pigment disorders and/or skin pigment imperfections, wherein said skin pigment disorders and/or skin pigment imperfections are associated with hyperpigmentation. The present invention also aims at additionally improving skin tone as well as providing a skin lightening or whitening agent.

### Background of the invention

Skin color is primarily determined by the amount and type of melanin, a brown pigment present in the skin. Lower amounts of melanin result in lighter skin color while higher amounts result in darker skin color. Also, hyper-pigmentation in the skin is caused by the over expression or accumulation of melanin in the skin. As a result, the pathway involved in melanin production has been the target for many inhibitors so as to reduce the levels produced. One of the principal enzymes involved in the melanin pathway is tyrosinase.

The synthesis of melanin is a process under hormonal control, including the melanocyte stimulating hormone (MSH) and adrenocorticotropic hormone (ACTH) peptides that are produced from the precursor proopiomelanocortin. It is stimulated by the DNA damages that are caused by UVB-radiations as well.

Then, exposure to the sun over time can induce many biochemical reactions in the skin, leading to sunburn and tanning, for example. Other consequences of exposure to the sun accumulate over time. These changes can result in the development of age spots and create an uneven, mottled skin tone. Unfortunately, many of the commercially available products in today's market are either only marginally effective, or contain active agents that are unstable and lose their potency when incorporated into a final formula.

The ability to modify the expression of pigment content in the skin, to promote an evenness skin tone or lightening skin tone, is highly desired in today's society. Many people desire to modify their skin tone, to reduce aging spots, etc., or for purely cosmetic reasons.

As a result, efforts to develop effective compositions have focused on agents that inhibit the activity of tyrosinase. For example, a variety of tyrosinase inhibitors, such as hydroquinone, vitamin C, cystein, kojic acid, arbutin and glutathione among others have been proposed in topical compositions. Also, a variety of dermatological compositions have been suggested for improving the appearance of pigment disorders such as that observed in melasma, freckles, vitiligo, piebaldism, phenylketonuria, and the like, and/or for cosmetic purposes.

Also, the use of skin bleaching compositions is widely expanded. However, they either destroy melanin or inhibit its formation. Many of these contain harsh chemicals such as peroxides, acids or formaldehyde, or thiolated materials. Less stringent therapies have other disadvantages.

Topical retinoid and topical corticosteroids have been suggested as hypo-pigmenting agents, as have laser treatment and chemical peels, but these fall short of desirable responses.

Other compositions suggested the use on the skin of natural materials, which have in some cases been used for centuries in Asia or Europe to bleach skin and skin areas, or enhance the appearance of fair skin. These include the use of lemon, orange, cucumber, ginkgo, carob, rose fruit, geranium herb, cinnamon, sweet marjoram, rosemary, etc...

In order to combat disorders related to abnormal pigment or to lighten skin tone, various compounds which, when applied topically to the skin, are capable of reducing tyrosinase activity and consequently limiting melanin production, have thus been proposed. Unfortunately, the treatments currently available are not entirely satisfactory, in particular in terms of the side effects which are frequently associated therewith, such as irritant side effects with certain topical agents.

It would thus be highly desirable to have alternative preparations that do not have the drawbacks of those described in the prior art. In particular, it would be highly desirable to develop nutritional cosmetic compositions to be administered via oral route that have improved stability and efficacy to promote an even skin tone or to lighten skin tone.

There also remains a need for active agents that are effective for treating and/or preventing skin pigmentation disorders, in particular those due to environmental factors or aging.

The object of the present invention is to meet these needs.

### Summary of the invention

The present inventors could achieve this object by providing a food or food supplement composition that comprises at least an ingredient containing green coffee beans treated with *Lactobacillus johnsonii* capable of hydrolysing chlorogenic acids to generate phenolic acids. This surprisingly resulted in an improved property on the regulation of skin pigmentation. Also, this treatment can take place in vivo when a human or an animal ingests a coffee extract in combination with *Lactobacillus johnsonii* capable of hydrolysing chlorogenic acids to generate phenolic acids.

Thus, according to a first subject, the invention relates to the cosmetic use of an effective amount of at least an ingredient containing green coffee portions with *Lactobacillus johnsonii* capable of hydrolysing chlorogenic acids to generate phenolic acids as an active agent for treating and/or preventing skin pigmentation disorders. Such skin disorder are in particular those due to age or to environmental factors (e.g. UV), such as age-spots. It may also be skin disorders that are observed in melasma, freckles and/or age spots.

The present inventors have discovered that treated green coffee beans effectively suppress the formation of melanin, melanogenesis, despite the fact that the extracts show little to no inhibition of tyrosinase activity. This result is surprising and unexpected considering the pivotal role of tyrosinase in melanogenesis and the focus of development efforts in the art to inhibit this enzyme.

For the purpose of the present invention, the term "skin" is intended to mean the skin of the face or of the body.

For the purpose of the present invention the term "effective amount", is intended to mean an amount sufficient to obtain the expected effect.

For the purpose of the present invention the term "prevent" is intended to mean the fact of reducing the risk of occurrence of the manifestation of the disorder under consideration.

The present invention is also directed towards the cosmetic use of the abovementioned ingredient, as an active agent for treating and/or preventing the skin pigment imperfections. As a result, the complexion becomes brighter and more homogeneous, without areas of dyschromia, or of dryness.

The present invention is also directed towards the cosmetic use of an effective amount of at least an ingredient containing green coffee beans according to the invention, as an active agent for whitening or lightening skin tone.

A use in accordance with the present invention may also comprise the use of at least an ingredient containing green coffee beans, in combination with an effective amount of at least one active agent for further improving skin hydration or skin ageing, in particular as described hereinafter.

According to another of its aspects, the subject of the invention is a method, in particular a cosmetic method, for treating and/or preventing skin tone imperfections and the disorders associated with hyper-pigmentation, in particular aesthetic disorders, in an individual, comprising at least one step of administering, to said individual, at least an ingredient containing green coffee beans according to the invention.

Compositions according to the present invention are orally administrable. This has the advantage of acting globally on the entire skin, in its deep layers (dermis, hypodermis), by means of a rapid and relatively non-restrictive mode of administration. Specifically, the metabolites and other active nutriments are in particular distributed within the dermal matrix by means of the bloodstream. Oral administration also has the advantage of a rapid and relatively non-restrictive mode of administration.

### Detailed description of the invention

According to a first object, the present invention provides the use of green coffee beans with a *Lactobacillus johnsonii* capable of hydrolysing chlorogenic acids to generate phenolic acids.

Chlorogenic acids are a family of esters formed between trans-cinnamic acids and quinic acid. Chlorogenic acids are naturally present in coffee, mainly as mono- and di-esters of quinic acid and phenolic groups (e.g. caffeic, ferulic, coumaric, methoxycinnamic) attached to different positions.

In one embodiment of the invention the microorganism is capable of hydrolysing caffeoyl quinic acid and diesters (e.g. 3-, 4-, or 5-caffeoyl quinic acid and diesters), and/or feruloyl quinic acid and diesters (e.g. 3-, 4-, or 5-feruloyl quinic acid and diesters), to generate caffeic acid and ferulic acid, respectively.

The composition of the invention should be formulated such that the microorganism will not ferment or react with the composition during storage. This may be achieved e.g. by formulating the composition as a dry powder, and/or by encapsulating the microrganism and/or enzyme so that the microorganisms and/or enzyme will only be released when the composition is mixed with coffee extract or during digestion.

### Coffee extract

A coffee extract according to the invention is an extract of green coffee beans by water or steam. Numerous methods for producing coffee extracts are known in the art. In particular, a coffee bean extract can be obtained by an aqueous, aqueous- alcoholic or alcoholic extraction of coffee beans, and preferably by an extraction with the aid of methanol, ethanol or propanol.

The green coffee may be derived from Arabica or Robusta, or may be a blend of Arabica and Robusta. The green coffee portion may be at least partially decaffeinated. This is to provide products the caffeine level of which can be modulated from caffeine-free to regular caffeine level.

### Microorganisms

Microorganisms capable of hydrolysing chlorogenic acid may e.g. be identified as disclosed in the examples of this application. The suitable microorganism is a Lactobacillus, being L. johnsonii (CNCM I-1225).

The microorganism should be present in an amount sufficient for hydrolysing a substantial amount of chlorogenic acids present in the coffee extract to phenolic acids during digestion. Preferably at least 20%, such as at least 30%, at least 50%, or at least 75% of caffeoyl quinic acids (CQA) and/or feruloyl quinic acids (FQA) present in the coffee extract is hydrolysed.

The compositions according to the invention may be in any of the galenical forms normally available for the method of administration selected. The carrier may be of diverse nature depending on the type of composition under consideration. Accordingly, the composition may be any food or pharmaceutical product, or a cosmetic product for oral application. Examples for food or pharmaceutical carriers nutritional complete formula, dairy product such as milk, yogurt, cheese, fermented milks, milk-based fermented products, ice-creams, cereal-based products or fermented cereal-based products, milk-based powders, infant and baby formulas, chilled or shelf stable beverages, food products of confectionary, chocolate or cereal type, animal feed, in particular for domestic animals. In particular, the chicory or extract thereof may be incorporated into any other form of food supplement or enriched food, for example food bars or compacted or non-compacted powders. The powders may be diluted in water, soda, milk products or soya bean derivatives, or be incorporated into food bars.

The food product may also further comprise a protein source, a carbohydrate source, a lipid source, a mineral source and/or a vitamin source. The presence of proteins, carbohydrates, lipids, minerals and/or vitamins may have several advantages. These compounds generally contribute to the taste and mouthfeel of the final product. They also provide the body with nutrients that it may need urgently when it is affected by skin disorders. They also allow formulating the product of the present invention as a complete nutritional formula, so that no additional nutrition is needed.

The individual ingredients may comprise any kind of edible compound. Typical ingredients for food compositions, in particular drinks, are well known in the art, e.g. milk, cream, coffee whiteners, and coffee creamers. Alternatively, ingredients could also be a salad dressing or parts thereof, for example. Such compounds are used by consumers to modify e.g. the aroma, appearance and texture of a composition. The ingredients may be in liquid or dry form, e.g. as powders, that are dissolved and/or suspended in a drink.

The invention also provides a coffee beverage product, such as (a) a roast and ground coffee product, (b) a soluble coffee product, (c) a single portion coffee pad, tablet or capsule (d) a ready to drink coffee product or (e) any other suitable coffee product, comprising the coffee product as defined herein as an ingredient.

Soluble coffee or instant coffee as it is otherwise known, is very well known and has been produced commercially for many decades. It is produced from roast and ground coffee in a variety of ways, all of which are well known to the person skilled in the art.

The present invention is particularly suitable for use as a soluble coffee product or any product, which is based on or derived from a soluble coffee product. For instance, the coffee product may also be used in so-called ready-to-drink beverages. Examples of ready-to-drink beverages for which the product of the present invention are suited include two-in-one beverages which comprise a coffee component together with a natural or artificial sweetener component, the components optionally being pre-diluted with a liquid such as water or milk. In this case, the coffee component can comprise the coffee product described herein.

Three-in-one beverages, which comprise coffee, a sweetener and a whitener such as milk, a liquid creamer or a solid (e.g. powdered) creamer, may comprise the coffee product described herein.

Furthermore, any beverage, which comprises soluble coffee as an ingredient, may comprise the coffee product described herein.

The composition may further comprise further ingredient suitable for inclusion in a food composition. Usual ingredients may e.g. be sugars, artificial sweeteners, emulsifiers, stabilizers, thickeners, flowing agents, colors, flavors, aromas, and the like. Suitable artificial sweeteners include saccharin, cyclamates, acetosulfame, L-aspartyl based sweeteners such as aspartame, and mixtures of these. Suitable emulsifiers include monoglycerides, diglycerides, lecithin, diacetyl tartaric acid esters of mono-diglycerides, emulsifying starches, and mixtures thereof. Suitable stabilisers include dipotassium phosphate and sodium citrate. A suitable flowing agent is sodium silica aluminate. In one embodiment the composition comprises milk protein and/or vegetable protein. In a further embodiment the composition comprises milk fat and/or vegetable fat.

Additionally, the the product described herein can be used as an ingredient for standard brew, espresso, coffee-based beverage preparations to be used as bulk or in single beverage portion for in-home preparation.

If the product is a nutritional supplement for oral administration it may be present in capsules, gelatin capsules, soft capsules, tablets, sugar-coated tablets, pills, pastes or pastilles, gums, or drinkable solutions or emulsions, a syrup or a gel. Such a supplement might also include a sweetener, a stabilizer, an antioxidant, an additive, a flavoring agent and/or a colorant. The formulation thereof is carried out by means of the usual methods for producing sugar-coated tablets, gel capsules, gels, hydrogels for controlled release, emulsions, tablets or capsules. In this respect the adjuvant for oral compositions, in particular for dietary supplements, are known to the specialist. Mention may be made, among others and for purely illustrative purposes, of lubricants such as magnesium stearate, products for instantaneous solubilisation, gelling agents, thickeners, moisturizers, fatty and/or aqueous compounds, preservatives, texturizing, flavoring and/or coating agents, anti-oxidants and coloring materials usually used in foods.

The composition described herein may contain, in addition, lipids, polyphenols, taurine, other probiotic microorganisms, vitamins and/or oligo-elements. If probiotics are used, they may be included in a live form, semi-active or in a desactivated form, e. g., as a lyophilized powder.

A kit may be used, comprising at least two parts: a) a first part comprising a coffee extract; and b) a second part comprising a microorganism and/or an enzyme capable of hydrolysing chlorogenic acids to generate phenolic acids. The two parts are sold together for the preparation of a composition but are physically separated in the packing of the product. The final composition to be consumed is prepared by mixing the two parts shortly before consumption. If one or both parts are in a liquid form they may be mixed directly, optionally further liquid, e.g. water or milk, may be added. The two parts may also be mixed by dissolving or suspending them in a liquid, e.g. water or milk. When liquid is used this may be hot or cold depending on whether a hot or a cold beverage is desired. If hot liquid is used, it may preferably have a temperature which is not so high as to inactivate the microorganism and/or enzyme before ingestion of the composition.

The first part of the kit comprises a coffee extract. Preferrably, the first part is in a dry form, e.g. in the form of a powder. The coffee extract may e.g. be a spray dried or freeze dried green coffee extract. The first part may also be in a liquid form. Liquid coffee extracts are readily available e.g. as ready-to-drink coffee beverages. The first part may additionally comprise any other suitable ingredient, e.g. chicory extract, aroma additives, stabilisers, salts, and/or sweeteners. The first part may be packed in any suitable way, e.g. in a sachet, bottle or can.

The second part comprises a microorganism and/or an enzyme capable of hydrolysing chlorogenic acids to generate phenolic acids. This part may preferably be in the form of a composition to be mixed with a coffee extract as described herein, preferably in the form of a coffee whitener or coffee creamer. It may comprise any other suitable components. It may be in dry form, e.g. as a powder, or in liquid form, and may be packed in any suitable way, e.g. in a sachet, bottle or can. It should be formulated such that the microorganism and/or enzyme will not ferment or react with other ingredients during storage. This may be achieved e.g. by formulating the composition as a dry powder, and/or by encapsulating the microrganism and/or enzyme so that the microorganisms and/or enzyme will only be released when the composition is mixed with coffee extract or during digestion.

The at least two parts may be packed together in any suitable way. They may e.g. be packed in a combined container wherein the parts are kept physically separated during storage and mixed when the container is opened, or they may be packed in separate containers which are sold together for the preparation of a beverage.

### Use

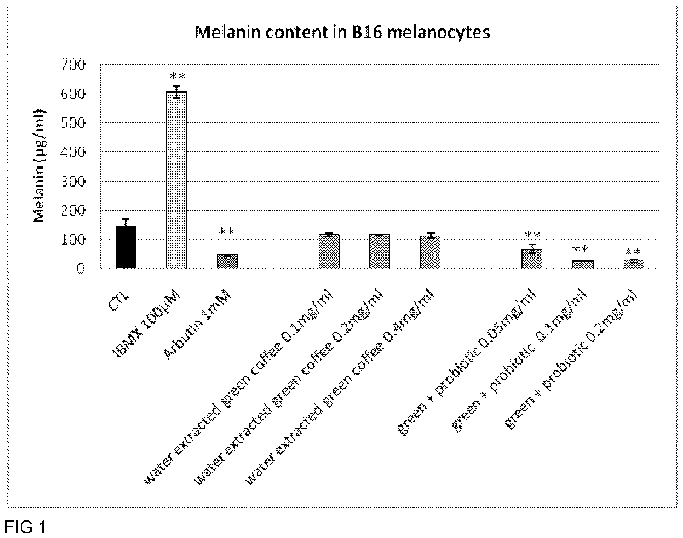

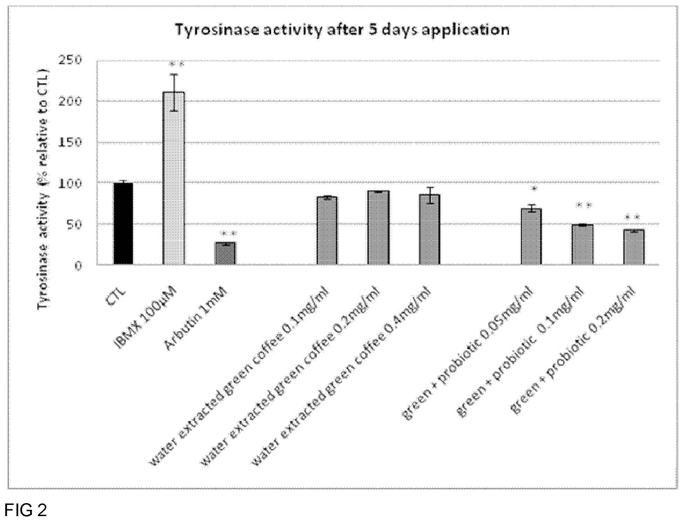

The products described herein are used for treating or preventing skin pigmentation disorders or cosmetically lightening skin tone e.g. by decreasing the production of melanin. Indeed green coffee beans extracts were shown to decrease in vitro the synthesis of melanin (Example 1, Figure 1). The production of tyrosinase was also decreased but to a limited extent (Figure 2), suggesting that the decrease in melanin was not due to tyrosinase inhibition but rather to mechanisms acting upstream or downstream of this enzyme.

The ingredients according to the present invention have further a positive effect on strengthening skin barrier and maintaining skin hydration.

As a result, the pigment imperfections are reduced, the complexion becomes brighter and more homogeneous, without areas of dyschromia, or of dryness.

Thus, according to one subject, the invention relates to the cosmetic use of an effective amount of at least one ingredient containing green coffee beans with *Lactobacillus johnsonii* capable of hydrolysing chlorogenic acids to generate phenolic acids for treating and/or preventing skin pigmentation disorders, in particular those due to age or environmental factors such as UV.

The present invention is also directed towards the cosmetic use of an effective amount of at least one ingredient containing green coffee beans with *Lactobacillus johnsonii* capable of hydrolysing chlorogenic acids to generate phenolic acids for whitening or lightening skin tone, which is particularly desirable for asian population.

A use in accordance with the present invention may also comprises the use of at least one ingredient containing green coffee beans with microorganisms capable of hydrolysing chlorogenic acids to generate phenolic acids, in combination with an effective amount of at least one active agent for improving skin hydration or skin ageing, in particular as described hereinafter.

According to another of its aspects, the subject of the invention is a method, in particular a cosmetic method, for treating and/or preventing skin tone imperfections and/or disorders associated with hyper-pigmentation, in particular aesthetic disorders, in an individual, comprising at least one step of administering, to said individual, at least one ingredient containing green coffee beans with *Lactobacillus johnsonii* capable of hydrolysing chlorogenic acids to generate phenolic acids according to the invention.

The cosmetic treatment method of the invention is carried out by orally administering at least an effective amount of at least one ingredient containing green coffee beans with *Lactobacillus johnsonii* capable of hydrolysing chlorogenic acids to generate phenolic acids in accordance with the invention. Oral administration comprises ingesting, in one or more intakes, an oral composition as defined above.

It may comprise a single application. According to another embodiment, the application is repeated, for example, 2 to 3 times a day, for one day or more, and generally for a sustained period of at least 4, or even 1 to 15, weeks.

In addition, combinations of treatment with, topical forms may be envisaged in order to supplement or reinforce the activity of the ingredients as defined by the invention.

Thus, an oral treatment with a composition containing green coffee beans in accordance with the invention, combined with a topical composition optionally containing another active ingredient, in particular a probiotic microorganism, or other probiotics in dead, live or semi-active form or an hydrating or anti-ageing agent could be imagined as a kit. The ingredients are mixed, before they are formulated, in the order and under conditions readily determined by those skilled in the art.

The proportion of coffee bean extract in the composition will of course be determined as a function of the desired effect and on the mode of administration of the composition.

The daily doses of green coffee bean extract administered by the oral route may preferably be comprised between 0.1 and 27 g/day depending on the type of product and the caffeine content, more preferably 0.5 to 18 g/day, even more preferably 1 to 3 g/day. The present inventors have found that the effectiveness of green coffee bean extracts according to the present invention is generally dose dependant and follows a dose response curve. If generally mild skin disorders or damages are to be prevented and the product will be used frequently, very small amounts will be sufficient to achieve the desired effect. If a severe skin pigment disorder is to be treated, larger amounts will be more appropriate, although also small amounts will produce an effect.

Preferably, the composition intended for oral administration contains an extract of coffee beans in a quantity ranging from 1% to 80% by weight of the composition and preferably from 10 % to 80% by weight of the composition depending on the product form.

Further advantages and features of the present invention are apparent from the following Examples and Figures. The examples hereinafter are thus presented by way of non-limiting illustration of the field of the invention. In these examples, unless otherwise indicated, the percentages are percentages by weight and the ranges of values written in the form "between ... and ..." include the upper and lower limits specified.

### Figures

**Figure 1**: Assessment of melanin production by murine melanocytes pre-treated with green coffee extracts.
**Figure 2****:** Assessment of tyrosinase production by murine melanocytes pre-treated with green coffee extracts.

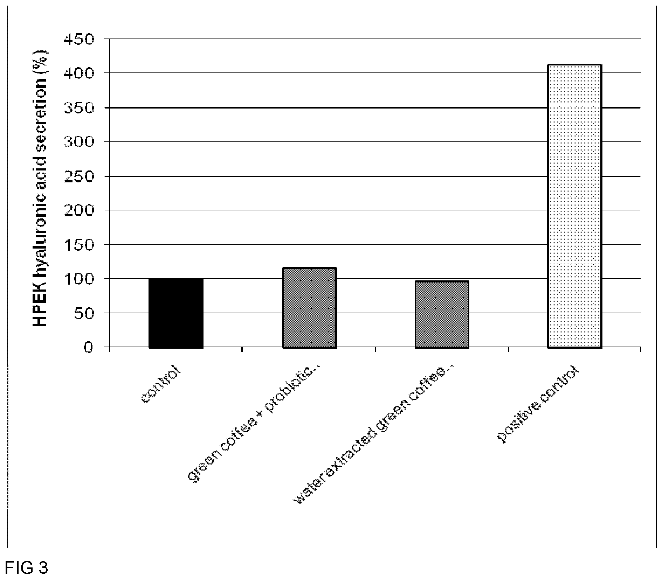

**Figure 3**: Assessment of hyaluronic acid secretion by human primary epidermal keratinocytes pre-treated with green coffee extracts.

### EXAMPLES

### Example 1

### Treatment of green coffee extract with a spray-dried preparation of Lactobacillus iohnsonii (CNCM 1-1225)

30 mg of a dried green coffee extract was dissolved in 1 ml phosphate buffer (50 mM, pH 7.0) or in 1 ml water. To this solution, 10 mg of a spray-dried preparation of Lactobacillus johnsonii (3.3 E9 cfu/g) was added. The mixture was then incubated at 37°C and samples were withdrawn at different reaction times. After centrifugation (3000 g, 5 min) and filtration (0.45 µm pore size syringe filters, Millipore SLHA 025 BS) the samples were analysed by HPLC.

### HPLC analysis

Coffee samples were diluted to 1% w/w and analyzed by RP-HPLC on a CC 250/4 Nucleosil 100-5-C18 column (Macherey-Nagel). The eluent system was Millipore water, 0.1% TFA and CH₃CN at a flow rate of 1 mL/min. The method allowed the simultaneous determination of caffeoyl quinic acids (CQA), feruloyl quinic acids (FQA), di-caffeoyl quinic acids (diCQA), feruloyl quinic acid-lactones, caffeic acid (CA) and ferulic acid (FA) (absorbance at 325 nm) using external standard calibration curves. Results were expressed relative to the reference at time 0 (t0) or to the reference at the same time without bacteria.

Results are shown in table 1.

**Table 1. Results of experiment 4. CQA, FQA, CA and FA are given as % relative to untreated control at t=0.**

| | | | | |
|---|---|---|---|---|
| Time (h) | 4 | 6 | 16 | 24 |
| CQA | 77 | 69 | 58 | 50 |
| FQA | 79 | 71 | 48 | 52 |
| diCQA | 67 | 53 | 32 | 20 |
| CA | 2673 | 3762 | 5182 | 6145 |
| FA | 961 | 1429 | 1963 | 2432 |

### Example 2 : Effect on skin pigmentation

In order to evaluate the potential beneficial effect of ingredients towards skin de- or pro-pigmentation we used 2D culture of murine melanocytes (B16) and we performed 2 tests: 1-assessment of melanin production and 2- assessment of tyrosinase production.

### 1. The cell culture conditions.

B16 cells were cultured in DMEM 1g/L glucose without phenol red supplemented with 10 % foetal calf serum, in a humidified chamber at 37 °C and containing 5% CO₂.

### 2. The production of melanin by B16 murine melanocyte cell line.

Cells were incubated with the selected ingredients or the test references (Kojic acid at 400µg/mL) for 72 hours, in the presence or absence of NDP-MSH an analog of MSH. The total quantity of melanin (extracellular and intracellular) was evaluated by measurement of the optical density at 405 nm of each sample against melanin standards in presence or in absence of NDP-MSH.

### 3. The production of tyrosinase by B16 murine melanocyte cell line.

Cells were incubated with the selected ingredients or the test references (Kojic acid at 400µg/mL) for 48 hours. The production of tyrosinase was evaluated by immunolabeling.

## Claims

1. Cosmetic, non-therapeutic use of an effective amount of at least green coffee beans or aqueous extracts thereof with *Lactobacillus johnsonii,* as an active agent for treating and/or preventing skin pigment disorders and/ or skin pigment imperfections, wherein said skin pigment disorders and/ or skin pigment imperfections are associated with hyper-pigmentation, wherein said active agent is comprised in an oral composition, and wherein *Lactobacillus johnsonii* is capable of hydrolysing chlorogenic acids to generate phenolic acids and does not ferment or react with the composition during storage.

2. Use in accordance with one of the preceding claims, **characterized in that** the skin pigment disorders are that observed in melasma, freckles, and/or age spots.

3. Use in accordance with one of the preceding claims for improving skin tone and/or skin complexion.

4. Use in accordance with one of the preceding claims for lightening and/ or whitening skin tone.

5. Use in accordance with one of the preceding claims, in which the extract is derived from coffee beans selected from the species Coffea arabica, Coffea robusta, Coffea canephora or Coffea iberica.

6. Use in accordance with one of the preceding claims, wherein the coffee beans are decaffeinated.

7. Use in accordance with one of the preceding claims, in which the coffee bean extract represents from 1% to 80% of the total weight of the composition.

8. Use according to any one of the preceding claims, in which said composition is a food product, a drink, a food supplement.

9. Cosmetic, non-therapeutic method for treating and/or preventing skin pigment disorders and/ or skin pigment imperfections, comprising at least one step of orally administering, to an individual, an effective amount of at least green coffee beans or an aqueous extract thereof with *Lactobacillus johnsonii,* wherein said skin pigment disorders and/ or skin pigment imperfections are associated with hyper-pigmentation, and wherein *Lactobacillus johnsonii* is capable of hydrolysing chlorogenic acids to generate phenolic acids and does not ferment or react with the composition during storage.

## Patentansprüche

1. Kosmetische, nichttherapeutische Verwendung einer wirksamen Menge von wenigstens grünen Kaffeebohnen oder wässrigen Extrakten davon mit *Lactobacillus johnsonii* als Wirkstoff zur Behandlung und/oder Vorbeugung von Hautpigmentstörungen und/oder Hautpigmentmakeln, wobei die Hautpigmentstörungen und/oder Hautpigmentmakel mit Hyperpigmentierung verbunden sind, wobei der Wirkstoff in einer oralen Zusammensetzung enthalten ist und wobei *Lactobacillus johnsonii* fähig ist, chlorogene Säuren zu hydrolysieren, um Phenolsäuren zu erzeugen, und die Zusammensetzung während der Lagerung nicht fermentiert oder damit reagiert.

2. Verwendung gemäß einem der vorstehenden Ansprüche, **dadurch gekennzeichnet, dass** die Hautpigmentstörungen in Melasma, Sommersprossen und/oder Altersflecken beobachtet werden.

3. Verwendung gemäß einem der vorstehenden Ansprüche zum Verbessern der Hautfarbe und/oder des Hautteints.

4. Verwendung gemäß einem der vorstehenden Ansprüche zum Aufhellen und/oder Bleichen der Hautfarbe.

5. Verwendung gemäß einem der vorstehenden Ansprüche, wobei der Extrakt aus Kaffeebohnen ausgewählt aus den Spezies Coffea arabica, Coffea robusta, Coffea canephora und Coffea iberica abgeleitet ist.

6. Verwendung gemäß einem der vorstehenden Ansprüche, wobei die Kaffeebohnen entkoffeiniert sind.

7. Verwendung gemäß einem der vorstehenden Ansprüche, wobei der Kaffeebohnenextrakt von 1 Gew.-% bis 80 Gew.-% des Gesamtgewichts der Zusammensetzung darstellt.

8. Verwendung gemäß einem der vorstehenden Ansprüche, wobei die Zusammensetzung ein Lebensmittelprodukt, ein Getränk oder eine Nahrungsergänzung ist.

9. Kosmetisches, nichttherapeutisches Verfahren zur Behandlung und/oder Vorbeugung von Hautpigmentstörungen und/oder Hautpigmentmakeln, umfassend wenigstens einen Schritt der oralen Verabreichung einer wirksamen Menge von wenigstens grünen Kaffeebohnen oder eines wässrigen Extrakts davon mit *Lactobacillus johnsonii* an eine Person, wobei die Hautpigmentstörungen und/oder Hautpigmentmakel mit Hyperpigmentierung verbunden sind und wobei *Lactobacillus johnsonii* fähig ist, chlorogene Säuren zu hydrolysieren, um Phenolsäuren zu erzeugen, und die Zusammensetzung während der Lagerung nicht fermentiert oder damit reagiert.

## Revendications

1. Utilisation cosmétique non thérapeutique d'une quantité efficace d'au moins un parmi des grains de café verts ou des extraits aqueux de ceux-ci avec *Lactobacillus johnsonii,* comme agent actif pour le traitement et/ou la prévention de troubles de pigmentation de la peau et/ou d'imperfections de la pigmentation de la peau, où lesdits troubles de pigmentation de la peau et/ou lesdites imperfections de la pigmentation de la peau sont associé(e)s à une hyper-pigmentation, où ledit agent actif est compris dans une composition orale, et où *Lactobacillus johnsonii* est capable d'hydrolyser des acides chlorogéniques afin de générer des acides phénoliques et ne fermente ni ne réagit avec la composition lors du stockage.

2. Utilisation selon l'une des revendications précédentes, **caractérisée en ce que** les troubles de pigmentation de la peau sont ceux observés avec le mélasme, les taches de rousseur et/ou le lentigo sénile.

3. Utilisation selon l'une des revendications précédentes, pour l'amélioration de la carnation de la peau et/ou du teint de la peau.

4. Utilisation selon l'une des revendications précédentes, pour l'éclaircissement et/ou le blanchiment de la carnation de la peau.

5. Utilisation selon l'une des revendications précédentes, dans laquelle l'extrait est dérivé de grains de café choisis parmi les espèces *Coffea arabica, Coffea robusta, Coffea canephora* ou *Coffea iberica.*

6. Utilisation selon l'une des revendications précédentes, **caractérisée en ce que** les grains de café sont décaféinés.

7. Utilisation selon l'une des revendications précédentes, **caractérisée en ce que** l'extrait de grains de café représente de 1% à 80% du poids total de la composition.

8. Utilisation selon l'une quelconque des revendications précédentes, **caractérisée en ce que** ladite composition est un produit alimentaire, une boisson ou un complément alimentaire.

9. Méthode cosmétique non thérapeutique destinée au traitement et/ou à la prévention de troubles de pigmentation de la peau et/ou d'imperfections de la pigmentation de la peau, comprenant au moins une étape d'administration par voie orale, à un individu, d'une quantité efficace d'au moins un parmi des grains de café verts ou un extrait aqueux de ceux-ci avec *Lactobacillus johnsonii,* où lesdits troubles de pigmentation de la peau et/ou lesdites imperfections de la pigmentation de la peau sont associé (e) s à une hyperpigmentation, et où *Lactobacillus johnsonii* est capable d'hydrolyser des acides chlorogéniques afin de générer des acides phénoliques et ne fermente ni ne réagit avec la composition lors du stockage.
